# EUROPEAN PATENT APPLICATION

(11) **EP 4 364 735 A1**
(43) Date of publication of application: **08.05.2024**
(21) Application number: 22831931.5
(22) Date of filing: 27.06.2022
(51) Int. Cl.: A61K 31/428, A61K 31/197, A61P 25/04

(54) **PHARMACEUTICAL COMPOSITION AND USE THEREOF**

(30) Priority: 01.07.2021 CN 202110742036
(71) Applicant: Neurodawn Pharmaceutical Co., Ltd., Jiangsu 211199 (CN)
(72) Inventor: LI, Fulong, Nanjing, Jiangsu 210046 (CN); ZHANG, Zhengping, Nanjing, Jiangsu 210046 (CN); FANG, Fang, Nanjing, Jiangsu 210046 (CN); YANG, Weidong, Nanjing, Jiangsu 210046 (CN); CHEN, Rong, Nanjing, Jiangsu 210046 (CN); YANG, Shibao, Nanjing, Jiangsu 210046 (CN)
(74) Representative: Lavoix
(86) International application number: PCT/CN2022/101423
(87) International publication number: WO 2023/274117

(57) **Abstract**

The present invention relates to a composition of (S)-3-aminomethyl-5-methylhexanoic acid or a pharmaceutically acceptable salt thereof, and riluzole. The composition has broad application prospects in the manufacture of a medicament for treating neuropathic pain.

## Description

This application claims the priority of Chinese Patent Application No. 202110742036.7, filed with the China National Intellectual Property Administration on July 1, 2021, and titled with "PHARMACEUTICAL COMPOSITION AND USE THEREOF", which are hereby incorporated by reference in entirety.

### FIELD

The present invention belongs to the pharmaceutical field, and relates to a composition of (S)-3-aminomethyl-5-methylhexanoic acid and riluzole, and use thereof in the manufacture of a medicament for the treatment of neuropathic pain.

### BACKGROUND

Neuropathic pain (NP) is pain caused by injury or disease of somatosensory system, which is divided into peripheral neuropathic pain and central neuropathic pain. Peripheral neuropathic pain is more common in clinical practice. A study from Europe shows that the prevalence rate of neuropathic pain in the general population is as high as 8.0%. Neuropathic pain is not a single disease, but a syndrome caused by different diseases and injuries, which manifests as a series of symptoms and signs, seriously affecting the quality of life of patients. Post-herpetic neuralgia and diabetic peripheral neuropathy are the two most common types of neuropathic pain. Chronic pain not only affects sleep, work and life ability of patients, but also increases the incidence of affective disorder, such as depression, anxiety, and the like. Some studies show that the score of life quality of patients with post-herpetic neuralgia is only 1/2 of that of the normal population. There is a lack of drugs to treat neuropathic pain. At present, calcium channel regulator, antidepressant and local anesthetics are mainly used to treat neuropathic pain in clinical practice. However, due to the complexity of pathogenesis of neuropathic pain, the performance of existing treatment is unsatisfactory, and a large proportion of patients fail to receive adequate relief of the pain.

In clinical practice, (S)-3-aminomethyl-5-methylhexanoic acid (pregabalin) can be used in the treatment of adult peripheral neuropathic pain, including diabetic peripheral neuralgia, fibromyalgia and post-herpetic neuralgia, and is recommended by many international guidelines as a first-line drug for the treatment of neuropathic pain. The mechanism of action of pregabalin is considered to regulate the voltage-gated calcium channel subunit α2δ and reduce the release of glutamic acid, noradrenaline and substance P. Pregabalin can cause dizziness, drowsiness and other adverse reactions, which limit its application to some extent in clinical practice.

The structural formula of (S)-3-aminomethyl-5-methylhexanoic acid (pregabalin) is as follows: 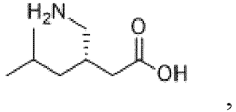 the molecular formula is C₈H₁₇NO₂, and the molecular weight is 159.23.

Riluzole (chemical name: 2-amino-6-trifluoromethyl benzothiazole) is a drug developed by Sanofi for the treatment of amyotrophic lateral sclerosis (ALS). It was approved for marketing by US FDA and EMA in 1996, and it plays a very important role in prolonging the survival period, relieving symptoms and improving the quality of life of patients with ALS. Its mechanism of action is thought to be related to inhibiting glutamic acid release, stabilizing the inactivated state of voltage-dependent sodium channel and interfering with intracellular events after the binding of neurotransmitter to excitatory amino acid receptor. Riluzole has a wide range of pharmacological effects, including regulation of glutamic acid and its transporter, antidepressant, anxiolytic, antiepileptic, analgesic and neuroprotective effect.

The structural formula of riluzole is as follows: 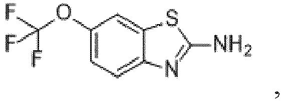 the molecular formula is C₈H₅F₃N₂OS, and the molecular weight is 234.20.

It should be pointed out that even two different drugs with the same pharmacodynamic effect are applied in combination, the efficacy of the combined application is very complicated and unpredictable, and may result in the phenomenon of synergy, addition and even antagonism of drugs. Therefore, for those skilled in the art, it is difficult to simply conclude that riluzole can enhance the analgesic effect of pregabalin by the fact of the analgesic effect of riluzole, unless necessary experimental data are obtained.

### SUMMARY

The present disclosure provides a pharmaceutical composition comprising (S)-3-aminomethyl-5-methylhexanoic acid and riluzole, which produces unexpected synergistic effect when used, and can enhance the therapeutic effect of resisting neuropathic pain.

The present disclosure provides use of a pharmaceutical composition in the manufacture of a medicament for treating neuropathic pain. The pharmaceutical composition comprises (S)-3-aminomethyl-5-methylhexanoic acid or a pharmaceutically acceptable salt thereof and riluzole or a pharmaceutically acceptable salt thereof.

In the composition, the mass ratio of (S)-3-aminomethyl-5-methylhexanoic acid or a pharmaceutically acceptable salt thereof to riluzole or a pharmaceutically acceptable salt thereof is 50:1 to 1:20 in free acid or base form.

Preferably, the mass ratio of (S)-3-aminomethyl-5-methylhexanoic acid or a pharmaceutically acceptable salt thereof to riluzole or a pharmaceutically acceptable salt thereof is 20:1 to 1:10 in free acid or base form.

Preferably, the mass ratio of (S)-3-aminomethyl-5-methylhexanoic acid or a pharmaceutically acceptable salt thereof to riluzole or a pharmaceutically acceptable salt thereof is 20:1 to 1:4 in free acid or base form.

Preferably, the mass ratio of (S)-3-aminomethyl-5-methylhexanoic acid or a pharmaceutically acceptable salt thereof to riluzole or a pharmaceutically acceptable salt thereof is 10:1 to 1:4 in free acid or base form.

Preferably, the mass ratio of (S)-3-aminomethyl-5-methylhexanoic acid or a pharmaceutically acceptable salt thereof to riluzole or a pharmaceutically acceptable salt thereof is 6:1 to 1:4 in free acid or base form.

Preferably, the mass ratio of (S)-3-aminomethyl-5-methylhexanoic acid or a pharmaceutically acceptable salt thereof to riluzole or a pharmaceutically acceptable salt thereof is 3:1 to 1:2 in free acid or base form.

The pharmaceutical composition of the present disclosure can be used for preparing a medicament for treating neuropathic pain, wherein the neuropathic pain is preferably peripheral neuropathic pain.

More preferably, the peripheral neuropathic pain is post-herpetic neuropathic pain and diabetic peripheral neuropathy.

### Beneficial effect:

The result of animal pharmacodynamic experiments shows that the pharmaceutical composition containing (S)-3-aminomethyl-5-methylhexanoic acid and riluzole provided by the present disclosure can play a synergistic effect and enhance the therapeutic effect of resisting peripheral neuropathic pain.

### DETAILED DESCRIPTION

The embodiments of the present disclosure will be clearly and completely described below in conjunction with the examples of the present disclosure. Obviously, the described examples are only a part of the embodiments of the present disclosure, rather than all the embodiments. Based on the examples of the present disclosure, all of other embodiments, made by those skilled in the art without any inventive efforts, fall into the scope of protection of the present disclosure.

### Example 1 Study 1 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

| **Drug name** | **manufacturer** | **batch number** |
|---|---|---|
| pregabalin | Adamas Reagent, Ltd | P1472904 |
| riluzole | Shanghai Aladdin Bio-Chem Technology Co., LTD | F1502089 |

### 1.3 Experimental methods

### 1.3.1 Preparation of animal model of neuropathic pain

In this experiment, the model of neuropathic pain induced by separate L5 spinal nerve ligation (SNL) was adopted. Animals were anesthetized with 7% chloral hydrate (dosage: 420 mg/kg, intraperitoneal injection), and the limbs of rats were fixed in a prone position. The rats were placed under dissecting microscope with the back hair-cut off, then wiped and disinfected with alcohol. An incision about 2-3 cm long was cut on the right side of the dorsal spine, and the two spinal nerves L4 and L5 were exposed, the spinal nerve L5 was lightly ligated with 6-0 braided line to avoid damage to the contiguous spinal nerves. Double-layer suture was performed on the back incisions of the experimental animals, and disinfected with iodophor, then the experimental animals were gently put into a feeding cage, allowed to free access to movement, eating and drinking.

### 1.3.2 Determination method of mechanical pain threshold

Mechanical withdrawal threshold (MWT) of all experimental animals was measured by mechanical automatic stimulated needle. The experimental animals were placed in a transparent plexiglass box with iron gauze at the bottom, and after acclimatization for 30 minutes, the vola of the hind limbs of the experimental animals were stimulated with a stimulated needle at a uniform speed, and the reaction threshold when rats rapidly retracted or shook their feet was recorded (as MWT) , which was the mechanical pain threshold of the animals.

### 1.3.3 Measurement and calculation of basic pain threshold of animals

The basic mechanical pain threshold of each experimental animal was measured for 2 days after modeling, and the basic mechanical pain threshold of experimental animals was the average of two values.

### 1.4 Animal grouping and experimental process

Experimental model animals were randomly divided into seven groups, namely model control group, pregabalin group (25 mg/kg), pregabalin group (0.5 mg/kg), riluzole group (10 mg/kg), riluzole group (0.5 mg/kg), a group of a composition with a ratio of pregabalin to riluzole of 50:1 (25 mg/kg pregabalin + 0.5 mg/kg riluzole) and a group of a composition with a ratio of pregabalin to riluzole of 1:20 (0.5 mg/kg pregabalin + 10 mg/kg riluzole). Pregabalin and riluzole were administered by intraperitoneal injection, and the model control group was administered with the corresponding blank solvent. Each group had 11-12 animals.

The L5-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h and 3h after administration.

### 1.5 Analysis of synergy of composition

According to Jin Zhengjun formula q= E(a+b)/(Ea+Eb-Ea×Eb), whether pregabalin and riluzole in the composition have synergistic effect was evaluated. In the formula, E(a+b) is the rate of improvement of the composition of two drugs, and Ea and Eb are the rates of improvement of drug A (pregabalin) and drug B (riluzole) administered alone, respectively. E = (pain threshold after administration-model value)/(basic value-model value). If the q value is in the range of 0.85- 1.15, it means that the combination of two drugs is simply additive; if the q value is greater than 1.15, it means that the combination of two drugs has a synergistic effect; if the q value is less than 0.85, it means that the combination of two drugs has an antagonistic effect.

### 1.6 Data statistics

The experimental data are expressed by mean± standard error (Mean± SEM). One-way ANOVA was used to analyze the differences between groups, and the comparison between groups was tested by LSD method, and P<0.05 was defined as significant difference.

### 2 Results

The experimental results are shown in Table 1. Compared with the solvent control group, pregabalin (25 mg/kg), riluzole (10 mg/kg), composition (50:1) and composition (1:20) can all significantly increase the mechanical pain threshold of animals at 0.5h after administration (P=0.0463, P<0.0001, P=0.0458, P<0.0001). All of the above groups can also significantly increase the mechanical pain threshold of the animals at 3h after administration (P<0.0001, P=0.0004, P<0.0001, P<0.0001). The calculation results of synergy showed that the q values of the composition(50:1) at 0.5h and 3h after administration were 1.073 and 0.919 respectively, and the q values of the composition(1:20) at 0.5h and 3h after administration were 0.909 and 0.998 respectively.

**Table 1 Mechanical pain threshold of animals in each group (unit: g)**

| **Group** | **number of animals** | **basic value** | **model value** | **pain threshold at 0.5h after administration** | **pain threshold at 3h after administration** |
|---|---|---|---|---|---|
| model control group | 11 | 27.04 ± 1.25 | 13.72 ± 0.29 | 14.26 ± 0.71 | 14.10 ± 0.99 |
| pregabalin group (25 mg/kg) | 11 | 29.68 ± 1.34 | 14.41 ± 0.54 | 17.41 ± 0.58* | 24.65 ± 0.34**** |
| pregabalin group (0.5 mg/kg) | 11 | 29.31 ± 1.21 | 14.35 ± 0.76 | 15.48 ± 1.27 | 15.54 ±1.15 |
| riluzole group (10 mg/kg) | 12 | 28.02 ± 1.11 | 13.73 ± 0.35 | 21.56 ± 1.24**** | 19.87 ± 0.92*** |
| riluzole group (0.5 mg/kg) | 12 | 28.75 ± 1.21 | 13.27 ± 0.44 | 14.25 ± 0.90 | 14.04 ± 0.91 |
| composition (50:1) group | 12 | 27.42 ± 1.07 | 13.71 ± 0.38 | 17.35 ± 1.35* | 23.15 ± 1.35**** |
| composition (1:20) group | 12 | 29.28 ± 1.16 | 13.95 ± 0.87 | 22.06 ± 1.11**** | 21.22 ± 1.46**** |

The data were expressed as Mean± SEM, *P<0.05, ***P<0.001, ****P<0.0001, compared with model control group.

### Example 2 Study 2 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

Pregabalin and riluzole were the same as in Example 1.

### 1.3 Experimental methods

The preparation of animal model of neuropathic pain, the measurement method of mechanical pain threshold and the measurement and calculation of basic pain threshold of animals were the same as in Example 1.

### 1.4 Animal grouping and experimental process

Experimental model animals were randomly divided into seven groups, namely model control group, pregabalin group (20 mg/kg), pregabalin group (1 mg/kg), riluzole group (10 mg/kg), riluzole group (1 mg/kg), a group of a composition with a ratio of pregabalin to riluzole of 20:1 (20 mg/kg pregabalin + 1 mg/kg riluzole) and a group of a composition with a ratio of pregabalin to riluzole of 1:10 (1 mg/kg pregabalin + 10 mg/kg riluzole). Pregabalin and riluzole were administered by intraperitoneal injection, and the model control group was administered with the corresponding blank solvent. Each group had 12 animals.

The LS-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h and 3h after administration.

### 1.5 Analysis of synergy of composition

Analysis of synergy of the composition was the same as in Example 1.

### 1.6 Data statistic

Data statistic wass the same as in Example 1.

### 2 Results

The experimental results are shown in Table 2. Compared with the solvent control group, pregabalin(20 mg/kg), riluzole(10 mg/kg), composition(20:1) and composition(1:10) can all significantly increase the mechanical pain threshold of animals at 0.5 h after administration (P=0.0278, P<0.0001, P=0.0483, P<0.0001). All of the above groups can also significantly increase the mechanical pain threshold of the animals at 3h after administration (P<0.0001, P<0.0001, P<0.0001, P<0.0001). The calculation results of synergy showed that the q values of the composition(20:1) at 0.5h and 3h after administration were 1.216 and 1.181 respectively, and the q values of the composition(1:10) at 0.5h and 3h after administration were 1.206 and 1.213 respectively. This showed that both of the composition (20:1) and the composition (1:10) had synergistic effect at 0.5h and 3h after administration.

**Table 2 Mechanical pain threshold of animals in each group (unit: g)**

| **Group** | **number of animals** | **basic value** | **model value** | **pain threshold at 0.5h after administration** | **pain threshold at 3h after administration** |
|---|---|---|---|---|---|
| model control group | 12 | 27.46 ± 1.11 | 13.74 ± 0.66 | 13.91 ± 0.68 | 14.24 ± 0.87 |
| pregabalin group (20 mg/kg) | 12 | 29.27 ± 1.18 | 14.52 ± 0.71 | 17.04 ± 0.54* | 24.76 ± 0.73**** |
| pregabalin group (1 mg/kg) | 12 | 29.38 ± 1.09 | 14.26 ± 0.68 | 15.57 ± 1.04 | 15.68 ± 1.32 |
| riluzole group (10 mg/kg) | 12 | 28.23 ± 1.17 | 14.05 ± 0.57 | 21.79 ± 1.33**** | 20.63 ± 1.12**** |
| riluzole group (1 mg/kg) | 12 | 28.47 ± 1.23 | 13.63 ± 0.61 | 14.08 ± 0.92 | 14.01 ± 0.99 |
| composition (20:1) group | 12 | 27.74 ± 1.31 | 13.26 ± 0.62 | 16.71 ± 1.21* | 25.27 ± 1.29**** |
| composition (1:10) group | 12 | 29.02 ± 1.37 | 13.42 ± 0.47 | 24.43 ± 0.95**** | 23.15 ± 1.12**** |

The data were expressed as Mean± SEM, *P<0.05, ****P<0.0001, compared with model control group.

### Example 3 Study 3 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

Pregabalin and riluzole were the same as in Example 1.

### 1.3 Experimental methods

The preparation of animal model of neuropathic pain, the measurement method of mechanical pain threshold and the measurement and calculation of basic pain threshold of animals were the same as in Example 1.

### 1.4 Animal grouping and experimental process

Experimental model animals were randomly divided into nine groups, namely model control group, pregabalin group (30 mg/kg), pregabalin group (18 mg/kg), pregabalin group (3 mg/kg), riluzole group (12 mg/kg), riluzole group (3 mg/kg), a group of a composition with a ratio of pregabalin to riluzole of 10:1 (30 mg/kg pregabalin + 3 mg/kg riluzole), a group of a composition with a ratio of pregabalin to riluzole of 1:4 (3 mg/kg pregabalin + 12 mg/kg riluzole) and a group of a composition with a ratio of pregabalin to riluzole of 6:1 (18 mg/kg pregabalin + 3 mg/kg riluzole). Pregabalin and riluzole were administered by intraperitoneal injection, and the model control group was administered with the corresponding blank solvent. Each group had 10-12 animals.

The L5-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h and 3h after administration.

### 1.5 Analysis of synergy of composition

Analysis of synergy of the composition was the same as in Example 1.

### 1.6 Data statistic

Data statistic was the same as in Example 1.

### 2 Results

The experimental results are shown in Table 3. Compared with the solvent control group, pregabalin(30 mg/kg), riluzole(12 mg/kg), composition(10:1), composition(1:4) and composition(6:1) can significantly increase the mechanical pain threshold of animals at 0.5 h after administration (P=0.0111, P<0.0001, P<0.0001, P<0.0001, P=0.003). Pregabalin(30 mg/kg), pregabalin(18 mg/kg), riluzole(12 mg/kg), composition(10:1), composition(1:4) and composition(6:1) can all significantly increase the mechanical pain threshold of the animals at 3h after administration (P<0.0001, P<0.0001, P<0.0001, P<0.0001, P<0.0001, P<0.0001). The calculation results of synergy showed that the q values of the composition (10:1) at 0.5h and 3h after administration were 1.273 and 1.182 respectively, the q values of the composition (1:4) at 0.5h and 3h after administration were 1.210 and 1.211 respectively, and the q values of the composition (6:1) at 0.5h and 3h after administration were 1.222 and 1.269 respectively. This showed that all of the composition (10:1), the composition (1:4) and the composition (6:1) had synergistic effect at 0.5h and 3h after administration.

**Table 3 Mechanical pain threshold of animals in each group (unit: g)**

| **Group** | **number of animals** | **basic value** | **model value** | **pain threshold at 0.5h after administration** | **pain threshold at 3h after administration** |
|---|---|---|---|---|---|
| model control group | 10 | 29.32 ± 1.26 | 14.27 ± 0.38 | 14.21 ± 0.44 | 14.57 ± 0.73 |
| pregabalin group (30 mg/kg) | 11 | 28.87 ± 1.52 | 14.89 ± 0.64 | 18.05 ± 0.73* | 26.13 ± 0.91 **** |
| pregabalin group (18 mg/kg) | 11 | 29.28 ± 1.44 | 14.16 ± 0.87 | 16.36 ± 1.35 | 23.92 ± 1.30**** |
| pregabalin group (3 mg/kg) | 11 | 29.22 ± 1.42 | 14.12 ± 0.49 | 15.19 ± 1.31 | 16.75 ± 1.14 |
| riluzole group (12 mg/kg) | 12 | 28.35 ± 1.29 | 14.36 ± 0.61 | 22.54 ± 1.33**** | 21.69 ± 1.12**** |
| riluzole group (3 mg/kg ) | 12 | 28.47 ± 1.23 | 13.23 ± 0.81 | 15.48 ± 0.92 | 15.16 ±1.31 |
| composition (10:1) group | 12 | 28.78 ± 1.17 | 13.73 ± 0.84 | 20.25 ± 1.12**** | 28.47 ± 1.38**** |
| composition (1:4) group | 12 | 29.73 ± 1.47 | 13.16 ± 0.64 | 25.47 ± 0.75**** | 25.34 ± 1.34**** |
| composition (6:1) group | 12 | 29.37 ± 1.44 | 13.29 ± 0.86 | 18.63 ± 0.48** | 27.38 ± 1.24**** |

The data were expressed as Mean± SEM, *P<0.05, **P<0.01, ****P<0.0001, compared with model control group.

### Example 4 Study 4 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

Pregabalin and riluzole were the same as in Example 1.

### 1.3 Experimental methods

The preparation of animal model of neuropathic pain, the measurement method of mechanical pain threshold and the measurement and calculation of basic pain threshold of animals were the same as in Example 1.

### 1.4 Animal grouping and experimental process

Experimental model animals were randomly divided into eleven groups, namely model control group, pregabalin group (18 mg/kg), pregabalin group (9 mg/kg), pregabalin group (3 mg/kg), riluzole group (12 mg/kg), riluzole group (6 mg/kg), riluzole group (3 mg/kg), a group of a composition with a ratio of pregabalin to riluzole of 6:1 (18 mg/kg pregabalin + 3 mg/kg riluzole), a group of a composition with a ratio of pregabalin to riluzole of 3:1 (9 mg/kg pregabalin + 3 mg/kg riluzole), a group of a composition with a ratio of pregabalin to riluzole of 1:4 (3 mg/kg pregabalin + 12 mg/kg riluzole) and a group of a composition with a ratio of pregabalin to riluzole of 1:2 (3 mg/kg pregabalin + 6 mg/kg riluzole). Pregabalin and riluzole were administered by intraperitoneal injection" and the model control group was administered with the corresponding blank solvent. Each group had 10 animals.

The L5-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h and 3h after administration.

### 1.5 Analysis of synergy of composition

Analysis of synergy of the composition was the same as in Example 1.

### 1.6 Data statistic

Data statistic was the same as in Example 1.

### 2 Results

The experimental results are shown in Table 4. Compared with the solvent control group, riluzole(12 mg/kg), riluzole(6 mg/kg), composition(6:1), composition(3:1), composition(1:4) and composition(1:2) can all significantly increase the mechanical pain threshold of animals at 0.5 h after administration (P<0.0001, P=0.0031, P=0.0033, P=0.0123, P<0.0001, P<0.0001). Pregabalin (18 mg/kg), pregabalin (9 mg/kg), riluzole (12 mg/kg), riluzole (6 mg/kg), composition (6:1), composition (3:1), composition (1:4) and composition (1:2) can all significantly increase the mechanical pain threshold of the animals at 3h after administration (P<0.0001, P<0.0001, P<0.0001, P=0.0427, P<0.0001, P<0.0001, P<0.0001, P<0.0001). The calculation results of synergy showed that the q values of the composition (6:1) at 0.5h and 3h after administration were 1.250 and 1.248 respectively, the q values of the composition (3:1) at 0.5h and 3h after administration were 1.389 and 1.391 respectively, the q values of the composition (1:4) at 0.5h and 3h after administration were 1.235 and 1.294 respectively, and the q values of the composition (1:2) at 0.5h and 3h after administration were 1.320 and 1.384 respectively. This showed that all of the composition (6:1), the composition (3:1), the composition (1:4) and the composition (1:2) had synergistic effect at 0.5h and 3h after administration.

**Table 4 Mechanical pain threshold of animals in each group (unit: g)**

| **Group** | **number of animals** | **basic value** | **model value** | **pain threshold at 0.5h after administration** | **pain threshold at 3h after administration** |
|---|---|---|---|---|---|
| model control group | 10 | 30.07 ± 1.33 | 14.91 ± 0.57 | 14.69 ± 0.72 | 14.38 ± 0.83 |
| pregabalin group (18 mg/kg) | 10 | 29.44 ± 1.26 | 14.32 ± 0.85 | 16.26 ± 0.74 | 24.54 ± 0.56**** |
| pregabalin group (9 mg/kg) | 10 | 29.36 ± 1.15 | 14.26 ± 0.83 | 15.03 ± 1.21 | 21.13 ± 1.13**** |
| pregabalin group (3 mg/kg) | 10 | 29.14 ± 1.23 | 14.43 ± 0.47 | 15.24 ± 0.95 | 16.96 ± 1.22 |
| riluzole group (12 mg/kg) | 10 | 28.35 ± 1.29 | 14.36 ± 0.61 | 22.54 ± 1.33**** | 21.69 ± 1.12**** |
| riluzole group (6 mg/kg) | 10 | 28.83 ± 1.27 | 13.32 ± 1.13 | 18.47 ± 0.37** | 17.47 ± 0.73* |
| riluzole group (3 mg/kg) | 10 | 27.91 ± 1.37 | 13.02 ± 1.06 | 15.43 ± 0.69 | 15.03 ± 0.79 |
| composition (6:1) group | 10 | 28.63 ± 1.18 | 13.28 ± 0.83 | 18.45 ± 1.22** | 27.07 ± 1.38**** |
| composition (3:1) group | 10 | 29.73 ± 1.47 | 13.16 ± 0.64 | 17.87 ± 0.74* | 25.34 ± 1.34**** |
| composition (1:4) group | 10 | 29.73 ± 1.44 | 13.29 ± 0.86 | 25.63 ± 0.56**** | 26.18 ± 1.25**** |
| composition (1:2) group | 10 | 28.28 ± 1.24 | 13.24 ± 0.49 | 20.56 ± 0.63**** | 21.43 ± 1.01**** |

The data were expressed as Mean± SEM, *P<0.05, **P<0.01, ****P<0.0001, compared with model control group.

### Example 5 Study 5 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

Pregabalin and riluzole were the same as in Example 1.

### 1.3 Experimental methods

The preparation of animal model of neuropathic pain, the measurement method of mechanical pain threshold and the measurement and calculation of basic pain threshold of animals were the same as in Example 1.

### 1.4 Animal grouping and experimental process

Experimental model animals were randomly divided into nine groups, namely model control group, pregabalin group (9 mg/kg), pregabalin group (6 mg/kg), pregabalin group (3 mg/kg), riluzole group (6 mg/kg), riluzole group (3 mg/kg), a group of a composition with a ratio of pregabalin to riluzole of 3:1 (9 mg/kg pregabalin + 3 mg/kg riluzole), a group of a composition with a ratio of pregabalin to riluzole of 1:2 (3 mg/kg pregabalin + 6 mg/kg riluzole) and a group of a composition with a ratio of pregabalin to riluzole of 1:1 (6 mg/kg pregabalin + 6 mg/kg riluzole). Pregabalin and riluzole were administered by intraperitoneal injection, and the model control group was administered with the corresponding blank solvent. Each group had 10-11 animals.

The L5-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h and 3h after administration.

### 1.5 Analysis of synergy of composition

Analysis of synergy of the composition was the same as in Example 1.

### 1.6 Data statistic

Data statistic was the same as in Example 1.

### 2 Results

The experimental results are shown in Table 5. Compared with the solvent control group, riluzole(6 mg/kg), composition(3:1), composition (1:2) and composition (1:1) can all significantly increase the mechanical pain threshold of animals at 0.5h after administration (P=0.0011, P=0.0006, P<0.0001, P<0.0001). Pregabalin (9 mg/kg), pregabalin (6 mg/kg), riluzole (6 mg/kg), composition (3:1), composition (1:2) and composition (1:1) can all significantly increase the mechanical pain threshold of the animals at 3h after administration (P<0.0001, P=0.0015, P=0.0315, P<0.0001, P<0.0001, P<0.0001). The calculation results of synergy showed that the q values of the composition (3:1) at 0.5h and 3h after administration were 1.362 and 1.384 respectively, the q values of the composition (1:2) at 0.5h and 3h after administration were 1.330 and 1.361 respectively, and the q values of the composition (1:1) at 0.5h and 3h after administration were 1.453 and 1.501 respectively. This showed that all of the composition (3:1), the composition (1:2) and the composition (1:1) had synergistic effect at 0.5h and 3h after administration.

**Table 5 Mechanical pain threshold of animals in each group (unit: g)**

| **Group** | **number of animals** | **basic value** | **model value** | **pain threshold at 0.5h after administration** | **pain threshold at 3h after administration** |
|---|---|---|---|---|---|
| model control group | 10 | 28.87 ± 1.25 | 13.38 ± 0.96 | 13.83 ± 0.99 | 14.04 ± 0.85 |
| pregabalin group (9 mg/kg) | 10 | 29.56 ± 1.42 | 14.29 ± 1.17 | 15.78 ± 0.83 | 21.66 ± 0.79**** |
| pregabalin group (6 mg/kg) | 10 | 29.20 ± 1.25 | 14.36 ± 0.41 | 15.24 ±1.15 | 19.15 ± 1.21 ** |
| pregabalin group (3 mg/kg) | 10 | 29.68 ± 1.46 | 14.25 ± 0.24 | 15.35 ± 0.57 | 16.73 ± 1.15 |
| riluzole group (6 mg/kg) | 10 | 28.36 ± 0.89 | 13.24 ± 1.24 | 18.21 ± 0.47** | 17.45 ± 0.46* |
| riluzole group (3 mg/kg) | 10 | 27.63 ± 1.28 | 13.35 ± 1.41 | 15.35 ± 0.58 | 14.98 ± 0.84 |
| composition (3:1) group | 11 | 28.36 ± 0.98 | 13.97 ± 0.60 | 18.36 ± 1.35*** | 24.76 ± 1.27**** |
| composition (1:2) group | 11 | 29.25 ± 1.74 | 13.42 ± 0.53 | 21.35 ± 0.85**** | 21.92 ± 1.35**** |
| composition (1:1) group | 11 | 28.92 ± 1.03 | 13.68 ± 0.49 | 21.84 ± 0.86**** | 25.38 ± 1.31**** |

The data were expressed as Mean ± SEM, *P<0.05, **P<0.01, ***P<0.001, ****P<0.0001, compared with model control group.

### Example 6 Study 6 on pharmacodynamic effect of the composition of pregabalin and riluzole on neuropathic pain

### 1 Materials and methods

### 1.1 Experimental animals

Sprague-Dawley(SD) rats, male, SPF grade, weighing 150-200g .

### 1.2 Drugs to be tested

Pregabalin and riluzole were the same as in Example 1.

### 1.3 Experimental methods

The preparation of animal model of neuropathic pain, the measurement method of mechanical pain threshold and the measurement and calculation of basic pain threshold of animals were the same as in Example 1.

### 1.4 Animal grouping and experimental process

Experimental model animals were randomly divided into eleven groups, namely model control group, pregabalin group (30 mg/kg), pregabalin group (20 mg/kg), pregabalin group (6 mg/kg), riluzole group (10 mg/kg), riluzole group (7.5 mg/kg), riluzole group (6 mg/kg), a group of a composition with a ratio of pregabalin to riluzole of 1:1 (6 mg/kg pregabalin + 6 mg/kg riluzole), a group of a composition with a ratio of pregabalin to riluzole of 2:1 (20 mg/kg pregabalin + 10 mg/kg riluzole), a group of a composition with a ratio of pregabalin to riluzole of 3:1 (30 mg/kg pregabalin + 10 mg/kg riluzole), a group of a composition with a ratio of pregabalin to riluzole of 4:1 (30 mg/kg pregabalin + 7.5 mg/kg riluzole) and a group of a composition with a ratio of pregabalin to riluzole of 5:1 (30 mg/kg pregabalin + 6 mg/kg riluzole). Pregabalin and riluzole were administered by intraperitoneal injection, and the model control group was administered with the corresponding blank solvent. Each group had 8-10 animals.

The L5-SNL model was prepared by selecting SD rats with appropriate weight, and the measurement of postoperative mechanical pain threshold was started at least 7 days after modeling. After operation, animals with significantly lower pain threshold of the right hind limb compared with the pain threshold of the opposite hind limb were selected for all subsequent efficacy tests. The experimental model animals were administered corresponding drugs or solvents, and the mechanical pain threshold of animals was measured at 0.5h, 3h, 6h and 8h after administration.

### 1.5 Analysis of synergy of composition

According to Jin Zhengjun formula q= E(a+b)/(Ea+Eb-Ea×Eb), whether pregabalin and riluzole in the composition have synergistic effect at Eₘₐₓ time point (3h) after administration was evaluated. In the formula, E(a+b) is the rate of improvement of the combination of two drugs, and Ea and Eb are the rates of improvement of drug A (pregabalin) and drug B (riluzole) administered alone, respectively. E = (pain threshold after administration-model value)/(basic value-model value). If the q value is in the range of 0.85- 1.15, it means that the combination of two drugs is simply additive; if the q value is greater than 1.15, it means that the combination of two drugs has a synergistic effect; if the q value is less than 0.85, it means that the combination of two drugs has an antagonistic effect.

CompuSyn software was used to analyzed whether the overall efficacy of pregabalin and riluzole in the composition had a synergistic effect within 0-8h after administration. The formula for calculating the improvement rate of drug group was (AUC₀₋₈ₕ- model value *8h)/ (basic value *8h- model value *8h). The combination index (CI) of each composition group was calculated by software. If CI<1, it means that the combination of two drugs has a synergistic effect , if CI=1, it means that the combination of two drugs is simply additive, and if CI>1, it means that the combination of two drugs has an antagonistic effect.

### 1.6 Data statistic

Data statistic was the same as in Example 1.

### 2 Results

The experimental results are shown in Table 6. Compared with the solvent control group, pregabalin (30 mg/kg), riluzole (10 mg/kg), riluzole (7.5 mg/kg), riluzole (6 mg/kg), a composition with a ratio of pregabalin to riluzole of 1:1, a composition with a ratio of pregabalin to riluzole of 2:1, a composition with a ratio of pregabalin to riluzole of 3:1, a composition with a ratio of pregabalin to riluzole of 4:1, and a composition with a ratio of pregabalin to riluzole of 5:1 can all significantly increase the mechanical pain threshold of animals at 0.5h after administration (P=0.018, P<0.0001, P<0.0001, P<0.0001, P<0.0001, P<0.0001, P<0.0001, P<0.0001, P<0.0001). Pregabalin (30 mg/kg), pregabalin (20 mg/kg), pregabalin (6 mg/kg), riluzole (10 mg/kg), composition (1:1), composition (2:1), composition (3:1), composition (4:1) and composition (5:1) can all significantly increase the mechanical pain threshold of the animals at 3h after administration (P<0.0001, P<0.0001, P=0.0008, P=0.023, P<0.0001, P<0.0001, P<0.0001, P<0.0001, P<0.0001). Pregabalin (30 mg/kg), pregabalin (20 mg/kg), pregabalin (6 mg/kg), composition (1:1), composition (2:1), composition (3:1), composition (4:1) and composition (5:1) can all significantly increase the mechanical pain threshold of the animals at 6h after administration (P<0.0001, P<0.0001, P=0.0007, P<0.0001, P<0.0001, P<0.0001, P<0.0001, P<0.0001). Pregabalin (30 mg/kg), pregabalin (20 mg/kg), pregabalin (6 mg/kg), composition (1:1), composition (2:1), composition (3:1), composition (4:1) and composition (5:1) can all significantly increase the mechanical pain threshold of the animals at 8h after administration (P<0.0001, P<0.0001, P=0.0008, P<0.0001, P<0.0001, P<0.0001, P<0.0001, P<0.0001). According to Jin Zhengjun formula, the synergy was calculated, and the results showed that the q value of the composition(1:1) at 3h after administration were 1.296, the q value of the composition (2:1) at 3h after administration were 1.258, the q value of the composition (3:1) at 3h after administration were 1.266, the q value of the composition (4:1) at 3h after administration were 1.319, and the q value of the composition (5:1) at 3h after administration were 1.264. This showed that all of the composition (1:1), the composition (2:1), the composition (3:1), the composition (4:1) and the composition (5:1) had synergistic effect at 3h after administration. CompuSyn software analysis showed that the CI value of AUC of the composition (1:1) after administration was 0.634, the CI value of AUC of the composition (2:1) after administration was 0.364, the CI value of AUC of the composition (3:1) after administration was 0.349, the CI value of AUC of the composition (4:1) after administration was 0.328, and the CI value of AUC of the composition (5:1) after administration was 0.349. This showed that all of the composition (1:1), the composition (2:1), the composition (3:1), the composition (4:1) and the composition (5:1) had synergistic effects within 0-8 hours after administration.

**Table 6 Mechanical pain threshold of animals in each group (unit: g)**

| **Group** | **number of animals** | **basic value** | **model value** | **pain threshold at 0.5h after administration** | **pain threshold at 3h after administration** | **pain threshold at 6h after administration** | **pain threshold at 8h after administration** |
|---|---|---|---|---|---|---|---|
| model control group | 8 | 28.46 ± 1.05 | 13.66 ± 0.82 | 13.78 ± 0.94 | 13.24 ± 0.79 | 13.86±0.93 | 14.08±1.11 |
| pregabalin group (30 mg/kg) | 8 | 29.27 ± 1.18 | 14.26 ± 0.97 | 18.65 ± 1.24* | 24.88 ± 0.96**** | 23.89±0.84**** | 21.83±1.07**** |
| pregabalin group (20 mg/kg) | 8 | 29.10 ± 1.17 | 14.82 ± 0.69 | 17.53 ± 1.33 | 23.52 ± 1.21**** | 22.35±1.14**** | 20.74±1.09**** |
| pregabalin group (6 mg/kg) | 8 | 27.88 ± 1.02 | 13.72 ± 0.85 | 17.39 ± 1.26 | 19.62 ± 1.13*** | 19.49±1.10*** | 19.26±0.94*** |
| riluzole group (10 mg/kg) | 9 | 28.58 ± 0.99 | 14.74 ± 1.08 | 24.45 ± 1.01**** | 17.89 ± 1.16* | 17.46±1.23 | 16.93±1.15 |
| riluzole group (7.5 mg/kg) | 9 | 29.16 ± 0.84 | 14.85 ± 1.03 | 23.93 ± 1.24**** | 16.44 ± 1.07 | 16.52±1.06 | 15.49±1.18 |
| riluzole group (6 mg/kg) | 9 | 27.87 ± 1.08 | 13.40 ± 0.68 | 22.96 ± 0.83**** | 15.74 ± 0.93 | 15.08±0.77 | 14.95±0.81 |
| composition (1:1) group | 8 | 28.94 ± 1.17 | 14.72 ± 0.88 | 21.93 ± 0.96**** | 24.14 ± 1.17**** | 22.97±0.85**** | 21.29±0.92**** |
| composition (2:1) group | 8 | 29.48 ± 1.08 | 15.23 ± 1.24 | 25.94 ± 1.19**** | 27.75 ± 1.04**** | 26.89±1.20**** | 25.27±0.79**** |
| composition (3:1) group | 9 | 29.40 ± 1.03 | 14.71 ± 1.05 | 25.28 ± 1.14**** | 29.11 ± 1.09**** | 25.99±1.13**** | 24.49±1.08**** |
| composition (4:1) group | 10 | 28.50 ± 1.12 | 15.20 ± 0.84 | 23.75 ± 0.86**** | 28.18 ± 0.95**** | 24.53±1.09**** | 23.64±1.05**** |
| composition (5:1) group | 9 | 27.98 ± 1.04 | 14.14 ± 0.70 | 22.32 ± 1.11**** | 27.34 ± 1.18**** | 22.68±1.04**** | 22.59±1.02**** |

The data were expressed as Mean ± SEM, *P<0.05, **P<0.01, **P<0.001, ****P<0.0001, compared with model control group.

## Claims

1. A composition for preparing a medicament for treating neuropathic pain, the composition comprises (S)-3-aminomethyl-5-methylhexanoic acid or a pharmaceutically acceptable salt thereof and riluzole or a pharmaceutically acceptable salt thereof.

2. The composition according to claim 1, wherein the mass ratio of (S)-3-aminomethyl-5-methylhexanoic acid or a pharmaceutically acceptable salt thereof to riluzole or a pharmaceutically acceptable salt thereof is 50:1 to 1:20 in free acid or base form.

3. The composition according to claim 1, wherein the mass ratio of (S)-3-aminomethyl-5-methylhexanoic acid or a pharmaceutically acceptable salt thereof to riluzole or a pharmaceutically acceptable salt thereof is 20:1 to 1:10 in free acid or base form.

4. The composition according to claim 1, wherein the mass ratio of (S)-3-aminomethyl-5-methylhexanoic acid or a pharmaceutically acceptable salt thereof to riluzole or a pharmaceutically acceptable salt thereof is 20:1 to 1:4 in free acid or base form.

5. The composition according to claim 1, wherein the mass ratio of (S)-3-aminomethyl-5-methylhexanoic acid or a pharmaceutically acceptable salt thereof to riluzole or a pharmaceutically acceptable salt thereof is 10:1 to 1:4 in free acid or base form.

6. The composition according to claim 1, wherein the mass ratio of (S)-3-aminomethyl-5-methylhexanoic acid or a pharmaceutically acceptable salt thereof to riluzole or a pharmaceutically acceptable salt thereof is 6:1 to 1:4 in free acid or base form.

7. The composition according to claim 1, wherein the mass ratio of (S)-3-aminomethyl-5-methylhexanoic acid or a pharmaceutically acceptable salt thereof to riluzole or a pharmaceutically acceptable salt thereof is 3:1 to 1:2 in free acid or base form.

8. Use of the composition according to any one of claims 1 to 7 in the manufacture of a medicament for the treatment of neuropathic pain.

9. The use according to claim 8, wherein the neuropathic pain is peripheral neuropathic pain.

10. The use according to claim 9, wherein the peripheral neuropathic pain is post-herpetic neuralgia.

11. The use according to claim 9, wherein the peripheral neuropathic pain is diabetic peripheral neuropathy.
